(19) Europäisches Patentamt
European Patent Office
Office européen des brevets

(11) Veröffentlichungsnummer: **0 269 042 B1**

# (12) EUROPÄISCHE PATENTSCHRIFT

(45) Veröffentlichungstag der Patentschrift: **16.10.91**

(51) Int. Cl.[5]: **C07C 67/465**, C07C 69/593

(21) Anmeldenummer: **87117208.6**

(22) Anmeldetag: **23.11.87**

## (54) Verfahren zur Herstellung von 2-Propenyliden-3-ethylglutarsäurediestern.

(30) Priorität: **27.11.86 DE 3640594**

(43) Veröffentlichungstag der Anmeldung:
**01.06.88 Patentblatt 88/22**

(45) Bekanntmachung des Hinweises auf die Patenterteilung:
**16.10.91 Patentblatt 91/42**

(84) Benannte Vertragsstaaten:
**BE DE FR GB IT NL**

(56) Entgegenhaltungen:

**JOURNAL OF ORGANIC CHEMISTRY, Band 46, 1981, American Chemical Society, S. 3795-3802; J. SHABTAI et al: "Base-catalyzed reactions of alpha,beta-unsaturated esters and nitriles. 4. Dimerization of beta-alkyl-substituted acrylates"**

**CHEMICAL ABSTRACTS, Band 67, Nr. 25, 18. Dezember 1967, Columbus, Ohio, USA, S. 10965, Spalte 1, Abstract Nr. 116522j; S. KYOICHI et al: "Dimerization of ethyl crotonate"**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**W-6900 Heidelberg(DE)**

**EP 0 269 042 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus J. Org. Chem. 46, Seiten 3795 ff. (1981) ist bekannt, daß man 2-Propenyliden-3-ethylglutarsäurediethylester durch Kondensation von 2-Pentensäureethylester bei 110 °C in Gegenwart von Katalysatorsystemen wie K-tert-Butylat, Kalium/Benzylkalium oder Natrium/Benzylnatrium erhält. Das Verfahren hat jedoch den Nachteil, daß bei der technischen Ausführung metallorganische Katalysatoren schwer handhabbar sind und zudem die erzielten Ausbeuten zu wünschen übrig lassen. Mit K-tert-Butylat sind jedoch die Umsätze gering. Es ist kein Hinweis zu entnehmen, wie die Ausbeuten gesteigert werden können.

Es war deshalb die technische Aufgabe gestellt, ein Verfahren zur Herstellung von 2-Propenyliden-3-ethylglutarsäurediestern zur Verfügung zu stellen, das mit höheren Ausbeuten verläuft und bei dem einfacher handhabbare Katalysatoren verwendet werden.

Diese Aufgabe wird gelöst in einem Verfahren zur Herstellung von 2-Propenyliden-3-ethylglutarsäurediestern der Formel I

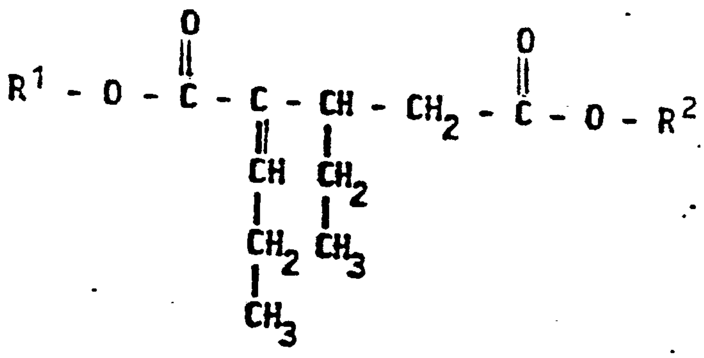

I,

in der $R^1$ und $R^2$ gleich oder verschieden sein können und jeweils einen Alkylrest mit 1 bis 12 Kohlenstoffatomen, einen Cycloalkylrest mit 5 bis 7 Kohlenstoffatomen, einen Aralkylrest mit 7 bis 10 Kohlenstoffatomen oder einen Arylrest mit 6 bis 10 Kohlenstoffatomen bedeutet durch Kondensation von Verbindungen der Formel II

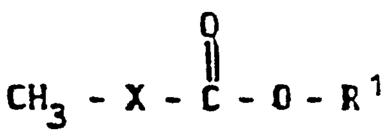

II,

in der X für die Reste

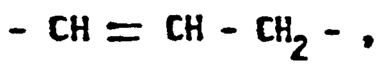

oder

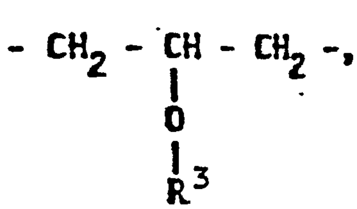

steht, wobei $R^3$ einen Alkylrest mit 1 bis 12 Kohlenstoffatomen bezeichnet und $R^1$ die obengenannte Bedeutung hat in Gegenwart von Katalysatoren bei erhöhter Temperatur, dadurch gekennzeichnet, daß man die Kondensation in Gegenwart von Alkoholaten von Alkali- oder Erdalkalimetallen, Aluminiumalkoholaten, Titanalkoholaten oder Amiden oder Hydriden von Alkali- oder Erdalkalimetallen oder cyclischen Amidinen

bei einer Temperatur von 100 bis 250 °C durchführt.

Das neue Verfahren hat den Vorteil, daß wenig aufwendige Katalysatoren eingesetzt werden. Ferner hat das neue Verfahren den Vorteil, daß man höhere Ausbeuten an 2-Propenyliden-3-ethylglutarsäurediestern erhält. Das neue Verfahren ist insofern bemerkenswert als 2-Pentensäureethylester in Gegenwart von Methanol und Natriummethylat 3-Methoxyvaleriansäuremethylester (Arkiv för Kemi, Band 12, Seiten 239 - 246 (1958) ergibt und bei der Behandlung von 2-Pentensäuremethylester mit Lithiumdiisopropylamid bei -70 °C 3-Pentensäuremethylester entsteht (E.-P. Krebs, Helv. Chimica Acta, Band 64, Seiten 1023 ff. (1981).

In den Ausgangsstoffen der Formel II steht der Substituent $R^1$ für einen Alkylrest mit 1 bis 12 Kohlenstoffatomen, einen Cycloalkylrest mit 5 bis 7 Kohlenstoffatomen, einen Aralkylrest mit 7 oder 8 Kohlenstoffatomen oder einen Arylrest mit 6 bis 10 Kohlenstoffatomen. Besonders bevorzugt sind Alkylreste mit 1 bis 8 Kohlenstoffatomen, insbesondere 1 bis 4 Kohlenstoffatomen. Ferner bezeichnet in Ausgangsstoffen der Formel II $R^3$ einen Alkylrest mit 1 bis 12 Kohlenstoffatomen, insbesondere 1 bis 4 Kohlenstoffatomen. Es versteht sich, daß die bevorzugten Endprodukte den bevorzugten Ausgangsstoffen entsprechen. Bei der Verwendung von gemischten Estern der Formel II hat $R^2$ die $R^1$ entsprechende Bedeutung in dem so erhaltenen Endprodukt der Formel I.

Geeignete Ausgangsstoffe der Formel II sind z.B. 3-Pentensäuremethylester, 3-Methoxyvaleriansäuremethylester, 3-Pentensäureethylester, 3-Pentensäure-iso-propylester, 3-Pentensäurecyclohexylester, 3-Pentensäurebenzylester, 3-Pentensäuredodecylester, 3-Propyloxyvaleriansäure-n-propylester oder Gemische derselben.

Die Kondensation wird in Gegenwart von Alkoholaten von Alkali- oder Erdalkalimetallen, Aluminumalkoholaten, Titanalkoholaten durchgeführt. Bevorzugte Alkoholate sind solche von Alkanolen mit 1 bis 4 Kohlenstoffatomen, insbesondere Alkali- oder Erdalkalimetallen.

Weitere geeignete Katalysatoren sind Amide oder Hydride von Metallen der 1. und 2. Gruppe, insbesondere von Natrium und Kalium sowie Calcium.

Geeignete Katalysatoren sind beispielsweise Natriummethylat, Natriumethylat, Kaliumethylat, Magnesiumethylat, Aluminiumbutylat, Titanbutylat, Natriumamid, Kaliumamid, Natriumhydrid, Kaliumhydrid oder Calciumhydrid.

Ferner sind geeignete Katalysatoren cyclische Amidine, insbesondere bicyclische Amidine, wie 1,5 Diazabicyclo[4,3,0]-nonen-5, 1,5-Diazabicyclo[5,4,0]-undecen-7, oder 1,4 Diazabcyclo[2,2,2]-octan.

Das Molverhältnis der Ausgangsverbindungen der Formel II zu den Katalysatoren beträgt vorteilhaft 1 : 0,01 bis 0,3, insbesondere 1 : 0,05 bis 0,1. Die Kondensation führt man bei einer Temperatur von 100 bis 250 °C, insbesondere von 100 bis 160 °C durch. Sofern man cyclische Amidine als Katalysatoren verwendet, sind Temperaturen von 150 bis 220 °C bevorzugt.

Im allgemeinen arbeitet man ohne Mitverwendung von Lösungsmitteln. Zur besseren Verteilung des Katalysators ist es jedoch häufig zweckmäßig inerte Lösungmittel mitzuverwenden, insbesondere Alkanole mit 1 bis 4 Kohlenstoffatomen. Solche Lösungsmittel werden vorteilhaft vor oder während der Kondensation durch Destillation abgetrennt.

Die Kondensation wird kontinuierlich oder diskontinuierlich bei Atmosphärendruck oder unter schwach erhöhtem Druck, z.B. bis zu 10 bar, zweckmäßig in der Flüssigphase durchgeführt. Bei einer diskontinuierlichen Arbeitsweise löst man beispielsweise den obenangegebenen Katalysator unter Mitverwendung eines Lösungsmittels in der Ausgangsverbindung I. Unter Abdestillieren des Lösungsmittels wird das Reaktionsgemisch auf die vorgenannte Reaktionstemperatur erhitzt und bei dieser Temperatur, z.B. für 10 bis 120 Minuten, gehalten. Nach dem Abkühlen wird der Katalysator abgetrennt, z.B. durch Neutralisieren zerstört und die hierbei anfallenden Produkte abfiltriert. Die Reaktionsprodukte werden dann durch Destillation gewonnen. Nichtungesetzte Ausgangsverbindungen werden zweckmäßig zurückgeführt.

Die nach dem Verfahren der Erfindung erhältlichen 2-Propenyliden-3-ethylglutarsäurediester stellen wertvolle Zwischenprodukte dar, die daraus erhältlichen hydrierten Dicarbonsäuren eignen sich zur Herstellung von Polyamiden.

Das Verfahren nach der Erfindung sei an folgenden Beispielen veranschaulicht.

Beispiel 1

In einem 500-ml-Dreihalskolben mit aufgesetzter Kolonne wurden 228 g 3-Pentensäuremethylester bei Raumtemperatur mit einer Lösung von 5,4 g Natriummethylat in 64 g Methanol vermischt. Unter Rühren und Abdestillieren von Methanol wurde aufgeheizt, bis die Sumpftemperatur im Kolben 130 °C erreicht hatte. Anschließend wurde eine Stunde lang bei 130 °C nachgerührt. Nach dem Abkühlen wurde das Reaktionsgemisch fraktioniert destilliert. Hierbei wurden 184,1 g eines Gemisches aus 2-Propenyliden- und 2-(1-Propenyl)-3-ethylglutarsäuredimethylester vom Siedepunkt 90 - 102 °C/0,3 mbar (Ausbeute 80 % d.Th.)

und 28,3 g Destillationsrückstand erhalten.

Beispiel 2

Eine Lösung von 0,4 g Natriummethylat in 4,7 g Methanol und 16,9 g 3-Pentensäuremethylester wurde in einem Glasautoklaven 4 Stunden lang auf 135° C erhitzt. Nach dieser Zeit bestand das Reaktionsgemisch zu 60 % aus 2-Propenyliden-3-ethylglutarsäuredimethylester zu 15 % aus 3-Methoxy-valeriansäuremethylester, zu 7 % aus 3-, zu 13 % aus 2-trans- und zu 3 % aus 2-cis-Pentensäuremethylester.

Vergleichsbeispiel 1

In einem 500-ml-Dreihalskolben wurden 228 g 3-Pentensäuremethylester bei Raumtemperatur mit einer Lösung von 5,4 g Natriummethylat in 64 g Methanol vermischt, auf 65 bis 70° C erwärmt und 4,75 Stunden bei dieser Temperatur gerührt. Nach dem Abkühlen wurde das Natriummethylat mit Eisessig neutralisiert und das Methanol bei Normaldruck weitgehend abdestilliert. Der Rückstand wurde mit Wasser gewaschen und anschließend fraktionierend destilliert. Hierbei wurden 185,6 g 3-Methoxyvaleriansäuremethylester (63 % d.Th.) vom Siedepunkt 125 bis 126° C/304 mbar erhalten. Außerdem fielen 46 g Pentensäuremethylester-Gemisch (31 % 3-, 8 % 2-cis-, , 61 % 2-trans-Pentensäuremethylester) (20 % d.Th.), 20 g 2-Propenyliden--3-ethylglutarsäuredimethylester und 3,2 g Destillationsrückstand an.

Beispiele 3 und 4

Wurde nicht 3-Pentensäuremethylester (3-PSE), sondern 2-trans-Pentensäuremethylester (2-trans-PSE), 2-cis-Pentensäuremethylester (2-cis-PSE) oder 3-Methoxyvaleriansäuremethylester (3-MVM) unter den in Beispiel 1 angegebenen Bedingungen eingesetzt, so wurden nach fraktionierender Destillation die in Tabelle 1 angegebenen Ausbeuten an 2-Propenyliden-3-ethylglutarsäuredimethylester erzielt.

Tabelle 1

| Ausgangs-verbindung | Molverhältnis $Ester:CH_3OH:CH_3ONa$ | Diester-Ausbeute [% d. Th.] | Selektivität [%] |
|---|---|---|---|
| 2-trans-PSE | | 81 | 86 |
| 2-cis-PSE | 1 : 1 : 0,05 | 80 | 84 |
| 3-MVM | | | |

PSE = Pentensäuremethylester

MVM = Methoxyvalerialsäuremethylester

Beispiele 5 und 6

Wurde das in Beispiel 1 verwendete Natriummethylat durch gleiche Molmengen Natriumamid oder Natriumhydrid ersetzt und ohne Zusatz von Methanol unter den Bedingungen von Beispiel 1 umgesetzt und aufgearbeitet, so wurden die in Tabelle 2 angegebenen Ausbeuten aus 2-Propenyliden-3-ethylglutarsäuredimethylester erzielt.

Tabelle 2

| Ausgangsverbindung | Katalysator | Molverhältnis 3-PSE : Kat. | Diester-Ausbeute [% d.Th.] | Selektivität [%] |
|---|---|---|---|---|
| 3-PSE | NaH | 1 : 0,05 | 80 | 84 |
| | $NaNH_2$ | | 71 | 84 |

Beispiel 7

Eine Lösung von 12,4 g 1,8-Diazabicyclo[4.3.0]nonen-5 (DBN) in 114 g 3-Pentensäuremethylester wurde in einem Glasautoklaven 2,5 Stunden lang auf 180 °C erhitzt. Das erhaltene Reaktionsgemisch (1124,3 g) wurde fraktionierend destilliert. Hierbei wurden zunächst 14 g eines Pentenester-Isomerengemisches erhalten, das zu 4 % aus 2-cis-PSE, zu 32 % aus 3-PSE und zu 53 % aus 2-trans-PSE bestand. Anschließend wurden 74,6 g eines Diester-Gemisches isoliert, das laut NMR-Analyse zu 71 % aus 2-Propenyliden und zu 29 % aus 2-(1-Propenyl)-3-ethylglutarsäuredimethylester bestand. Außerdem wurden 34 g Rückstand isoliert. Die Diester-Ausbeute betrug 61 % d.Th.

**Patentansprüche**

1. Verfahren zur Herstellung von 2-Propenyliden-3-ethylglutarsäurediestern der Formel I

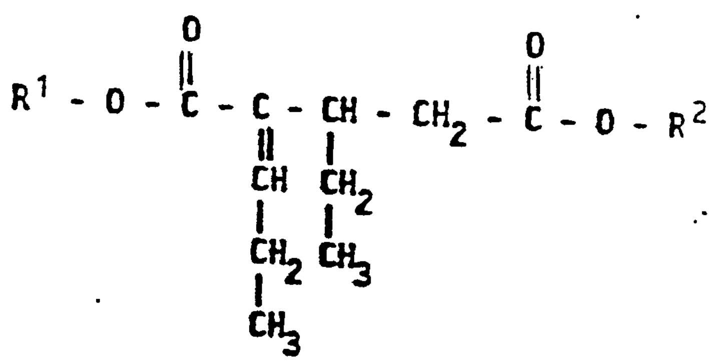

I,

in der $R^1$ und $R^2$ gleich oder verschieden sein können und jeweils einen Alkylrest mit 1 bis 18 Kohlenstoffatomen, einen Cycloalkylrest mit 5 bis 7 Kohlenstoffatomen, einen Aralkylrest mit 7 oder 8 Kohlenstoffatomen oder einen Arylrest mit 6 bis 10 Kohlenstoffatomen bezeichnen durch Kondensation von Verbindungen der Formel II

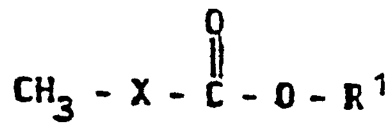

II,

in der X für die Reste

$-CH=CH-CH_2-$,

oder

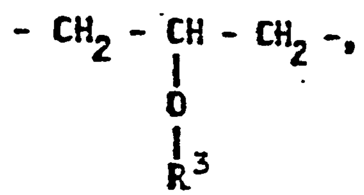

steht, wobei $R^3$ einen Alkylrest mit 1 bis 12 Kohlenstoffatomen bezeichnet und $R^1$ die vorgenannte Bedeutung hat, in Gegenwart von Katalysatoren bei erhöhter Temperatur, dadurch gekennzeichnet, daß man die Kondensation in Gegenwart von Alkoholaten von Alkali- oder Erdalkalimetallen, Aluminiumalkoholaten, Titanalkoholaten oder Amiden, oder Hydriden von Alkali- oder Erdalkalimetallen oder cyclischen Amidinen bei einer Temperatur von 100 bis 250° C durchführt.

2. Verfahren nach Anspruch 1, dadurch gekennzeichnet, daß man 3-Pentensäure $C_1$- bis $C_4$-Alkylester oder 3-Alkoxyvaleriansäure $C_1$- bis $C_4$-Ester als Ausgangsstoffe verwendet.

3. Verfahren nach den Ansprüchen 1 und 2, dadurch gekennzeichnet, daß man Alkoholate von Alkalimetallen mit $C_1$- bis $C_4$-Alkanolen, Alkalihydride oder Alkaliamide als Katalysatoren verwendet.

## Claims

1. A process for preparing 2-propenylidene-3-ethylglutaric diesters of the formula I

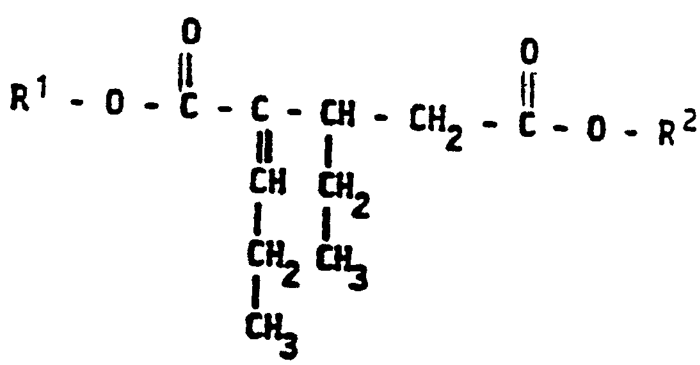

I,

where $R^1$ and $R^2$ can be identical or different and each is alkyl of 1 to 18 carbons, cycloalkyl of 5 to 7 carbons, aralkyl of 7 or 8 carbons or aryl of 6 to 10 carbons, by condensation of compounds of the formula II

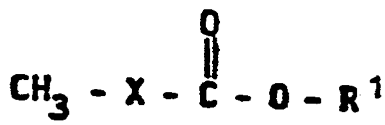

II,

where X is

$- CH = CH - CH_2 -$,

or

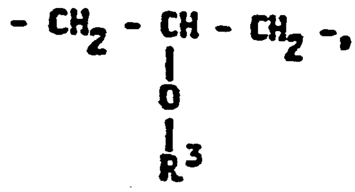

where $R^3$ is alkyl of 1 to 12 carbons, and $R^1$ has the abovementioned meaning, in the presence of catalysts at elevated temperature, which comprises carrying out the condensation in the presence of alcoholates of alkali metals or alkaline earth metals, aluminum alcoholates, titanium alcoholates, or amides, or hydrides of alkali metals or alkaline earth metals or cyclic amidines at from 100 to 250 °C.

2. A process as claimed in claim 1, wherein $C_1$-$C_4$-alkyl esters of 3-pentenoic acid or $C_1$-$C_4$-alkyl esters of 3-alkoxyvaleric acid are used as starting materials.

3. A process as claimed in claims 1 and 2, wherein alcoholates of alkali metals with $C_1$-$C_4$-alkanols, alkali metal hydrides or alkali metal amides are used as catalysts.

**Revendications**

1. Procédé de préparation de diesters d'acide 2-propénylidéne-3-éthylglutarique de formule I

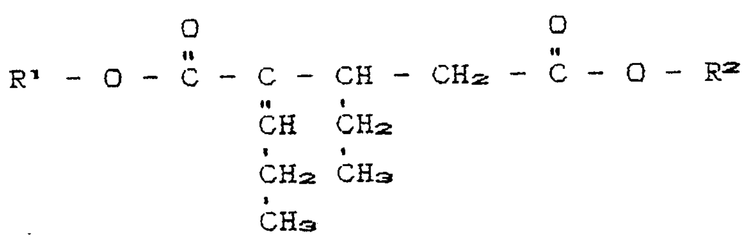

I

dans laquelle $R^1$ et $R^2$ peuvent être identiques ou différents et représentent chacun un reste alkyle à 1-18 atomes de carbone, un reste cycloalkyle à 5-7 atomes de carbone, un reste aralkyle à 7 ou 8 atomes de carbone ou un reste aryle à 6-10 atomes de carbone, par condensation de composés de formule

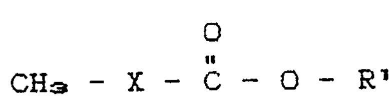

II

dans laquelle X est mis pour les restes

$$- CH = CH - CH_2 -$$

ou

$$- CH_2 - \underset{\substack{| \\ O \\ | \\ R^3}}{CH} - CH_2 -,$$

$R^3$ représentant un reste alkyle à 1-12 atomes de carbone et $R^1$ ayant la signification donnée précédemment, en présence de catalyseurs et à température élevée, caractérisé en ce qu'on conduit la condensation en présence d'alcoolates de métaux alcalins ou alcalino-terreux, d'alcoolates d'aluminium, d'alcoolates de titane, d'amidures ou d'hydrures de métaux alcalins ou alcalino-terreux ou d'amidines cycliques, à une température de 100 à 250 °C.

2. Procédé selon la revendication 1, caractérisé en ce qu'on utilise, comme substances de départ, des esters alkyliques en $C_1$-$C_4$ d'acide 3-penténoique ou des esters alkyliques en $C_1$-$C_4$ d'acide 3-alcoxyvalérique.

3. Procédé selon la revendication 1 ou 2, caractérisé en ce qu'on utilise, comme catalyseurs, des alcoolates de métaux alcalins avec des alcanols en $C_1$-$C_4$,des hydrures alcalins ou des amidures alcalins.